(19) **Europäisches Patentamt**
**European Patent Office**
**Office européen des brevets**

(11) **EP 1 862 148 B1**

(12) **EUROPEAN PATENT SPECIFICATION**

(45) Date of publication and mention
of the grant of the patent:
**07.10.2009 Bulletin 2009/41**

(51) Int Cl.:
*A61F 2/16* (2006.01)

(21) Application number: **07108531.0**

(22) Date of filing: **21.05.2007**

(54) **Intraocular lenses with enhanced off-axis visual performance**

Intraokuläre Linsen mit erweiterter außeraxialer visueller Leistung

Lentilles intraoculaires avec performance visuelle désaxée améliorée

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **31.05.2006 US 443766**

(43) Date of publication of application:
**05.12.2007 Bulletin 2007/49**

(73) Proprietor: **Alcon, Inc.**
**6331 Hünenberg (CH)**

(72) Inventors:
• **Hong, Xin**
**Arlington, TX 76017 (US)**

• **Karakelle, Mutlu**
**Fort Worth, TX 76132 (US)**
• **Zhang, Xiaoxiao**
**Fort Worth, TX 76132 (US)**

(74) Representative: **Hanna, Peter William Derek et al**
**Hanna Moore & Curley**
**13 Lower Lad Lane**
**Dublin 2 (IE)**

(56) References cited:
**EP-A- 0 329 981          WO-A-02/084381**
**WO-A-20/05098518      US-A- 1 741 947**
**US-A- 5 895 422**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention is generally directed to ophthalmic lenses, and more particularly, to intraocular lenses (IOLs) that provide enhanced on-axis and off-axis visual performance.

### BACKGROUND

Intraocular lenses are routinely implanted in patients' eyes during cataract surgery to replace the natural crystalline lens. A variety of aberrations, such as spherical aberrations or comatic aberrations, can adversely affect the visual performance of such implanted IOLs. For example, spherical aberrations can degrade vision contrast, especially for large pupil sizes. Some conventional IOLs provide correction for a single aberration, e.g., spherical aberration, but do not address the issue of multiple aberrations.

In optical imaging systems, such as IOLs, light from an object in the center of a viewing field is focused at a focal point defined by the optics. The focus, however, is wavelength dependent. Hence, while light at the design wavelength can be focused at the focal point, light at other wavelengths will be focused either in front or behind the ideal focal point. This type of "on-axis" aberration is known as chromatic aberration.

Off-axis aberrations are also common in optical systems. In cases of "spherical" aberration, light from the objects in the periphery of the viewing field are focused either in front or behind the ideal focal point. In cases of "coma," the images of peripheral objects may also be somewhat unfocused and instead appear wedge-shaped. The further off-axis, the worse this effect appears and hence, the name "comatic aberration" or coma, since it was first recognized in viewing stars with telescopes.

Spherical aberration, like chromatic aberrations, is a radially symmetrical form of aberration, while coma is an asymmetric aberration. Another form of asymmetric aberration is "trefoil," in which three distinct axes with different curvatures are present. Each of these forms of aberration (as well as others) can be present in ophthalmic lenses, especially when they form part of the total vision system, including the patient's cornea.

Accordingly, there is a need for enhanced ophthalmic lenses, and more particularly, for enhanced IOLs that can compensate for multiple aberrations.

WO 02/084381 (Pharmacia Groningen BV) describes an ophthalmic lens comprising a diffractive part and a method for designing such a lens. According to the invention the ophthalmic lens further comprises a refractive part comprising at least one surface, which is configured to compensate a passing wavefront at least partly for at least one type of monochromatic aberration introduced by at least one of the optical parts of the eye. Said diffractive part is capable of compensating a passing wavefront at least partly for chromatic aberration introduced by at least one of the optical parts of the eye. Furthermore said refractive and diffractive parts together contribute to a required power of the lens.

### SUMMARY

The present invention is directed generally to multi-surface and/or multi-element intraocular lenses (IOLs) in accordance with claims which follow, in which a plurality of surfaces are adapted to provide compensation for a variety of aberrations, and in particular, for off-axis aberrations such as coma, or trefoil, in addition to on-axis aberrations, such as spherical aberration. In some embodiments, different surfaces are adapted to compensate for different aberrations so as to provide enhanced on-axis as well as off-axis visual performance. By way of example, an aberration value, which can be defined as root-mean-square (RMS) of the aberration, can be measured over a 6 mm apparent (or entrance) pupil when the lens is implanted in a human eye (or a model eye), which can correspond to a lens aperture size of about 5 mm for an ophthalmic lens implanted in the human capsular bag. Unless otherwise indicated, the aberration values recited herein are based on these criteria, and hence, for ease of description, the RMS definition and the 6 mm qualification will be omitted in connection with the aberration values recited in the sections that follow.

In one aspect, an intraocular lens is disclosed that includes a posterior optic and an anterior optic. One of the optics provides compensation for a radially symmetric aberration and the other provides compensation for a radially asymmetric aberration. As used herein, an optic provides compensation for an aberration by completely or partially correcting (counteracting) the effects of that aberration. For example, when the aberration causes an axial spread of the focal point, the compensation can decrease the spread so as to generate a sharper focus.

In a related aspect, the radially symmetric aberration comprises spherical aberration and the radially asymmetric aberration comprises any of coma or trefoil. In some cases, at least one of the posterior or anterior optics can be adapted to provide compensation in a range of about - 0.5 (minus 0.5) microns to about +0.5 microns (plus 0.5 microns) for an aberration. By way of example, each optic can include at least one optical surface whose base profile exhibits a selected degree of asphericity (departure from a spherical surface) designed to counteract an aberration, e.g., spherical aberration.

In another aspect, the posterior and anterior optics are axially separated by a distance in a range of about 0 to about 5 millimeters. In many cases, the posterior and anterior optics are disposed relative to one another such that their optical axes are substantially aligned.

In another aspect, the posterior and anterior optics collectively provide an optical power in a range of about 6 Diopters to about 34 Diopters. The optics are preferably formed of biocompatible materials, such as soft acrylic, silicone, hydrogel or other biocompatible polymeric materials having a requisite index of refraction for a particular application. While in some cases both optics are formed of the same material, in others, they can be formed of different materials.

In a related aspect, the anterior and posterior optics have different chromatic dispersions (variations of index of refraction as a function of wavelength) so as to cooperatively provide compensation for chromatic aberrations.

In another aspect, an intraocular lens is disclosed that includes an optic having a posterior optical surface and an anterior optical surface. The anterior surface is adapted to provide compensation for a radially symmetric aberration and the posterior surface is adapted to provide compensation for a radially asymmetric aberration. By way of example, the radially symmetric aberration comprises spherical aberration while the radially asymmetric aberration comprises any of coma or trefoil.

In a related aspect, one of the posterior or the anterior surfaces includes an aspheric, symmetric base profile that provides compensation for spherical aberration, e.g., by providing a correction in a range of about - 0.5 (minus 0.5) microns to about +0.5 (plus 0.5) microns, while the other surface includes an asymmetric profile adapted to provide compensation for coma, and/or trefoil, e.g., by providing a correction in a range of about - 0.5 microns to about +0.5 microns.

The intraocular lens can be formed of a biocompatible material, and can be adapted to provide an optical power in a range of about 6 Diopters to about 34 Diopters.

In another aspect, the invention provides an intraocular lens (IOL) that includes a posterior optic and an anterior optic, wherein the posterior optic comprises at least one optical surface adapted to provide compensation for one aberration type and the anterior optic comprises at least one optical surface adapted to provide compensation for another aberration type.

In a related aspect, one of the aberration types can comprise a radially symmetric aberration, e.g., spherical aberration, while the other aberration type can comprise a radially asymmetric aberration, e.g., coma.

In another aspect, at least one of the posterior or anterior optics comprises another optical surface adapted to provide compensation for a third aberration type, e.g., trefoil.

Further understanding of the invention can be obtained by reference to the following detailed description in conjunction with the drawings, which are described briefly below.

<u>BRIEF DESCRIPTION OF THE DRAWINGS</u>

FIGURE 1 is a cross-sectional view of a multi-element IOL according to one embodiment of the invention,

FIGURE 2 is another cross-sectional view of the IOL of FIGURE 1 schematically illustrating an asphericity associated with the anterior surface of the anterior optic of the IOL,

FIGURE 3 is another cross-sectional view of the IOL of FIGURE 1 schematically illustrating an asymmetry imparted to an anterior surface of the IOL's posterior optic for correcting coma, and

FIGURE 4 is a schematic cross-sectional view of an IOL in accordance with another embodiment of the invention comprising an optic having an anterior surface shaped to provide compensation for a radially symmetric aberration (e.g., spherical aberration) and a posterior surface shaped to provide compensation for a radially asymmetric aberration (e.g., coma).

<u>DETAILED DESCRIPTION</u>

The present invention relates generally to multi-element and/or multi-surface ophthalmic lenses in which different elements and/or surfaces provide independent correction of a plurality of monochromatic, polychromatic and oblique aberrations. In the embodiments that follow, the salient features of various aspects of the invention are discussed in connection with intraocular lenses (IOLs). However, the teachings of the invention can also be applied to other ophthalmic lenses, such as contact lenses. Further the term "intraocular lens" and its abbreviation "IOL" are used herein interchangeably to describe lenses that are implanted into the interior of the eye to either replace the eye's natural lens or to otherwise augment vision regardless of whether or not the natural lens is removed. Intracomeal lenses and phakic lenses are examples of lenses that may be implanted into the eye without removal of the natural lens.

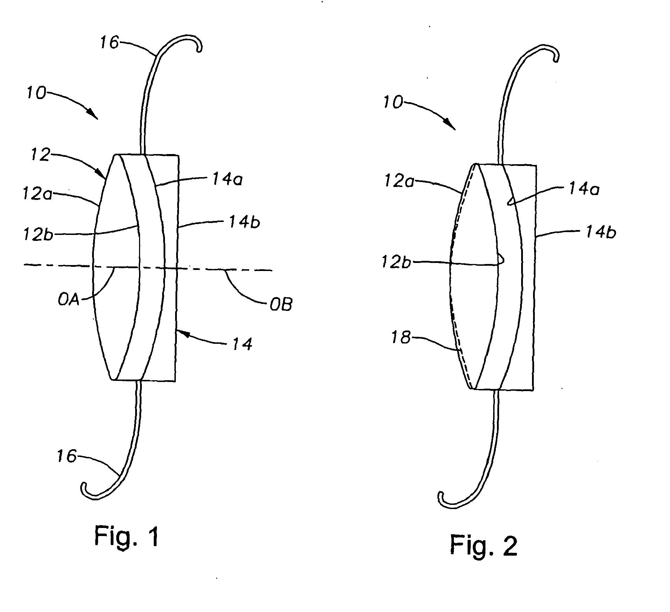

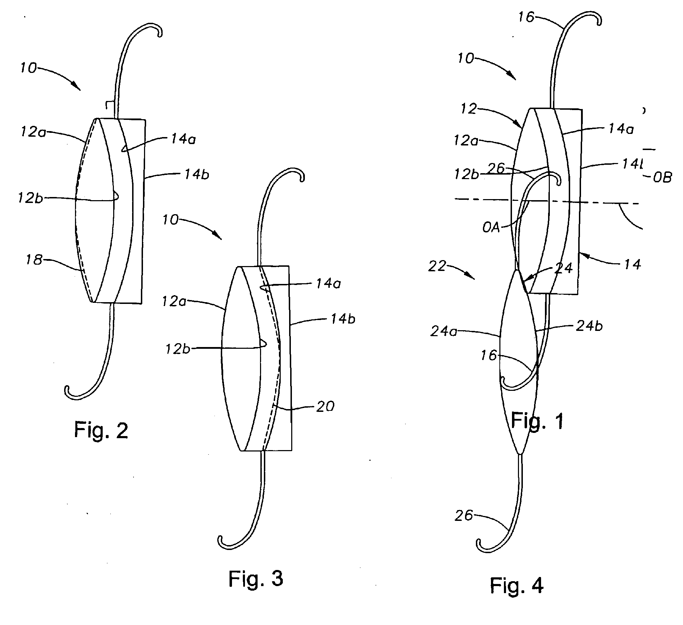

With reference to FIGURE 1, an exemplary intraocular lens (IOL) 10 according to one embodiment of the invention includes an anterior optic 12 and a posterior optic 14. The optic 12 can be characterized by an optical axis OA and the optic 14 can be characterized by an optical axis OB. In many embodiments, the optical axes OA and OB are substantially aligned.

In some embodiments, one or more surfaces of at least one optic, and/or the optic itself, can be asymmetric relative to the respective optical axis, e.g., to reduce off-axis aberrations as discussed further below. Although in this embodiment the optics 12 and 14 are axially separated from one another, in other embodiments, the optics can be in contact via two surfaces thereof More generally, in many embodiments, the separation between the optics can range from zero to about 5 mm. The IOL 10 further includes fixation members or haptics 16 that facilitate its placement in a patient's eye.

In many embodiments, the anterior and posterior optics collectively provide an optical power in a range of about 6 Diopters (D) to about 34 D. Further, the optics are preferably formed of biocompatible materials, such as soft acrylic, silicone, hydrogel or other biocompatible polymeric materials having a requisite index of refraction for a particular application. By way of further examples, U.S. Patent No. 6,416,550, discloses materials suitable for forming the IOL 10. The haptics 16 can also be formed of suitable polymeric materials, such as polymethylmethacrylate, polypropylene and the like.

While in some embodiments, both optics are formed of the same material, in other embodiments, they can be formed of different materials. By way of example, in this exemplary embodiment, the posterior optic can be formed of a soft acrylic material known as Acrysof® (a cross-linked copolymer of 2-phenylethyl acrylate and 2-phenylethyl methacrylate) having an index of refraction of about 1.55, while the anterior optic is formed of another material having a lower index of refraction (e.g., 1.42) so as to reduce surface reflections and glare.

With continued reference to FIGURE 1, anterior optic 12 includes an anterior surface 12a and a posterior surface 12b that provide the optic with a generally bi-convex shape. The posterior optic is, in turn, formed of a generally concave anterior surface 14a and a substantially flat posterior surface 14b. Other shapes can also be employed for the anterior and/or posterior optics, such as plano-convex.

One or more optical surfaces of the optics 12 and 14 are configured so as to reduce, and in some cases eliminate, a number of radially symmetric and radially asymmetric aberrations. By way of example, as shown schematically in FIGURE 2, in this embodiment, the anterior surface 12a of the anterior optic 12 exhibits an aspheric base profile that reduces spherical aberration - a radially symmetric aberration. That is, the anterior surface 12a includes a base profile that is substantially coincident with a putative spherical profile 18 (depicted by dashed lines) at small radial distances from the optical axis but exhibits an increasing deviation from that spherical profile as the radial distance from the optical axis increases. In some embodiments, the asphericity of the profile can be selected to provide a compensation in a range of about - 0.5 microns to about + 0.5 microns, and preferably in a range of about - 0.1 microns to about - 0.3 microns, for the spherical aberration.

In some embodiments, the aspherical profile of the anterior surface can be defined in accordance with the following relation:

$$z = \frac{cr^2}{1+[1-(1+k)c^2 r^2]^{\frac{1}{2}}} + a_1 r^2 + a_2 r^4 + a_3 r^6 \qquad \text{Eq. (1)}$$

wherein,

z denotes a sag of the surface at a radial distance r from an optical axis of the optic 12, c denotes curvature of the surface at its apex (at the intersection of the optical axis with the surface); $c = \frac{1}{R}$ where R denotes the radius of the surface at its apex,

k denotes a conic constant,
$a_1$ denotes a second order aspheric coefficient,
$a_2$ denotes a fourth order aspheric coefficient, and
$a_3$ denotes a sixth order aspheric coefficient.

In some embodiments, the aspheric profile of the anterior surface can be characterized by the above relation with c ranging from about 0.0152 mm$^{-1}$ to about 0.0659 mm$^{-1}$, k ranging from about -1162 to about -19, $a_1$ ranging from about -0.00032 mm$^{-1}$ to about - 0.00020 mm$^{-1}$, $a_2$ ranging from about - 0.0000003 (minus 3x10$^{-7}$) mm$^{-3}$ to about - 0.000053 (minus 5.3x10$^{-5}$) mm$^{-3}$, and $a_3$ ranging from about 0.0000082 (8.2x10$^{-6}$) mm$^{-5}$ to about 0.000153 (1.53x10$^{-4}$) mm$^{-5}$.

With continued reference to FIGURES 1 and 2, in this embodiment, the posterior optic 14 is shaped so as to provide compensation for a radially-asymmetric aberration, such as coma. For example, the profile of the anterior surface 14a of the posterior optic 14 can be adapted to provide compensation (e.g., in a range of about - 0.5 to about +0.5 microns, and preferably in a range of about - 0.35 to about + 0.35 microns) for coma. As known in the art, coma is an off-axial aberration that is non-symmetrical about the optical axis. Coma can arise, e.g., when light rays incident on a lens are not parallel to the lens's optical axis, thereby affecting the off-axis performance of the lens. The off-axis performance of an IOL implanted in a patient's eye can be important as the human eye depends on peripheral vision for, e.g., transient object perception. Further, patients who suffer from age-related macular degeneration (AMD) typically rely heavily on their peripheral vision to perform visual tasks. Hence, for such a patient having an implanted IOL, the off-axis performance of the IOL can be important.

More particularly, with reference to FIGURE 3, in this exemplary embodiment, the profile of the anterior surface 14a of the posterior optic 14 deviates from a putative spherical profile 20 (shown in dashed lines) in a rotationally asymmetric manner relative to the optical axis so as to reduce coma. In some embodiments, such asymmetric profile of the surface 14a can be defined in accordance with the following relation:

$$z = c_{coma} * f_{coma}(r,\theta,\alpha), \qquad \text{Eq. (2)}$$

wherein,

$$f_{coma}(r,\theta,\alpha) = 2\sqrt{3}(10r^5 - 12r^3 + 3r)\cos(\theta + \alpha) \qquad \text{Eq. (3)}$$

wherein,

z indicates a sag of the surface along the optical axis,
$C_{coma}$ is a coefficient indicating a correction magnitude (e.g., in a range of about - 0.5 microns to about + 0.5 microns),
r is a pupil location normalized relative to the pupil radius,
θ denotes a meridian angle, and
α represents the coma axis to be corrected.

Referring again to FIGURE 1, in another embodiment, the anterior optic 12 provides compensation for one or more radially asymmetric aberrations while the posterior optic provides compensation for a radially symmetric aberration. For example, the anterior surface 12a of the anterior optic can be adapted to compensate for coma, e.g., in a manner discussed above, while the profile of its posterior surface 12b can be adapted to compensate for another radially asymmetric aberration, such as trefoil. For example, the profile of the posterior surface 12b can be adapted to provide a compensation in a range of about - 0.35 to about +0.35 microns for the trefoild aberration. Further, the anterior surface 14a of the posterior optic 14 can provide a correction for a rotationally symmetric aberration (e.g., spherical aberration), for example, in a manner discussed above.

By way of example, in some embodiments, the profile of a surface of the lens, which provides a correction for the trefoil aberration, can be defined in accordance with the following relation:

$$z = c_{trefoil} * f_{trefoil}(r,\theta,\alpha) \qquad \text{Eq. (4)}$$

wherein,

$$f_{trefoil}(r,\theta,\alpha) = 2\sqrt{3}(5r^5 - 4r^3)\cos(3(\theta + \alpha)) \qquad \text{Eq. (5)}$$

wherein,

$C_{trefoil}$ is a coefficient indicating a correction magnitude (e.g., in a range of about - 0.5 microns to about +0.5 microns),
r is a pupil location normalized relative to the pupil radius,
θ is a meridian angle, and

$\alpha$ is the trefoil axis to be corrected.

In some embodiments, the chromatic dispersions (variations of refractive index as a function of wavelength) of the materials forming the optics 12 and 14 of the IOL 10, together with the radii of curvature of their optical surfaces, are selected to reduce, or substantially eliminate, the longitudinal chromatic aberrations exhibited by the IOL 10, and/or to provide compensation for the natural chromatic aberrations of the eye. For example, one optic (e.g., 12) can be configured to have a positive optical power and be made of one type of material and the other optic (e.g., 14) can be configured to have a negative optical power and be made of a different material such that the IOL would provide chromatic aberrations correction. For example, in some embodiments, the IOL can provide a chromatic aberration correction in a range of about 1 to about 2 Diopters over a wavelength range of about 400 nm to about 700 nm. As is known in the art, a variation of the refractive index of a material as a function of radiation wavelength is referred to as the dispersion of that material. One commonly employed measure of a material's dispersion (variation of refractive index with wavelength) is known as Abbe number (also known as V-number or constringence of a material), and is defined as follows:

$$V = \frac{n_D - 1}{n_F - n_C} \qquad \text{Eq. (6)}$$

where $n_D$, $n_F$ and $n_C$ represent the refractive indices of the material at wavelengths of 589.2 nm, 486.1 nm and 656.3 nm, respectively, that correspond to Fraunhofer D-, F-, and C-spectral lines. In general, materials having high values of V exhibit low dispersions. In some embodiments, the materials forming the optics 12 and 14 have sufficiently different V numbers so as to minimize, and in some cases eliminate the chromatic aberrations of the IOL.

By way of example, in one embodiment, the optic 12 can be made from polymethylmethacrylate (PMMA) (V = 55) and the optic 14 can be made from polysulfone (V = 30.87). Other suitable materials include, without limitation, soft acrylics (V of about 37), polystyrene (V = 30.87), polycarbonate (V = 29.9), or cellulose acetate hydrate (V in a range of about 80 to 84) so long as the differences between the Abbe numbers of the materials forming the two optics are sufficiently large (e.g., greater than about 10) to provide a desired chromatic compensation. Our European patent application no. 1,862,147 entitled "Correction of Chromatic Aberrations in Intraocular Lenses," provides further details regarding correcting chromatic aberrations in intraocular lenses.

The teachings of the invention are not limited to multi-optic ophthalmic lenses. In other embodiments, one surface of a single-optic lens is employed to compensate for a radially symmetric aberration while the other surface of that optic is utilized to compensate for a radially asymmetric aberration. By way of example, FIGURE 4 schematically illustrates an IOL 22 according to another embodiment of the invention that includes an optic 24 having an anterior surface 24a and a posterior surface 24b. The IOL 22 further includes a plurality of fixation members or haptics 26 that facilitate its placement in a patient's eye. Similar to the previous embodiments, the IOL 22 is preferably formed of a biocompatible material, such as those discussed above. Although in this embodiment, the IOL 22 has a bi-convex shape, in other embodiments, other shapes can be employed. In this embodiment, the anterior surface 24a has a surface profile that is adapted to compensate for a radially asymmetric aberration (e.g., coma or trefoil) while the posterior surface 24b exhibits a profile adapted to compensate for a radially symmetric aberration (e.g., spherical aberration). For example, the anterior surface can be characterized by the above Equations (1) while the posterior surface is characterized by the above Equations (2) and (3) or Equations (4) and (5).

The use of different optics of a multi-optic IOL and/or different surfaces of a single-optic IOL for compensation of a plurality of aberrations advantageously allows independent adjustment of a number of distinct aberration modes. Further, it can facilitate customizing the IOLs to suit the visual needs of individual patients by streamlining the manufacturing processes. For example, for each optical surface of the IOL, a series of optic pins with different correction amounts associated with a given aberration mode can be set up. A permutation of such optic pins corresponding to different surfaces can be employed to provide IOLs exhibiting compensation for different aberrations and/or different amounts of aberration correction.

Those having ordinary skill in the art will appreciate that various changes can be made to the above embodiments without departing from the scope of the invention.

**Claims**

1. A multi-element or multi-surface intraocular lens (10,22) (IOL), comprising:

a posterior optical surface (24b,14a,14b), and
an anterior optical surface (24a,12a,12b),
wherein at least one optical surface (24b,14a,14b) is adapted to provide compensation for one aberration type
and at least one optical surface (24a,12a,12b) is adapted to provide compensation for another aberration type
**characterized in that** a combination of;

the degree of asphericity of the optical surfaces;
the Abbe number of the lens material;
the radii of curvature of the optical surfaces;
the axial separation of the optical surfaces;
the degree of alignment of the optical axes of the optical surfaces,
are selected to cooperatively reduce, or substantially eliminate, the longitudinal chromatic aberrations exhibited by the intraocular lens (10), and/or to cooperatively provide compensation for the natural chromatic aberrations of the eye, whereby a plurality of monochromatic, polychromatic and oblique aberrations may be independently corrected and off-axis visual performance is enhanced by providing customized IOLs exhibiting compensation for different aberrations and/or different amounts of aberration correction.

2. The intraocular lens of claim 1, comprising:

a posterior optic (14), and
an anterior optic (12),
wherein said posterior optic comprises at least one optical surface (14a,14b) adapted to provide compensation for one aberration type and said anterior optic comprises at least one optical surface (12a,12b) adapted to provide compensation for another aberration type.

3. The intraocular lens of claim 1 or claim 2, wherein one of said aberration types comprises a radially symmetric aberration and the other aberration type comprises a radially asymmetric aberration.

4. The intraocular lens of claim 2, wherein one of said posterior and anterior optics (12,14) provides compensation for a radially symmetric aberration, and the other provides compensation for a radially asymmetric aberration.

5. The intraocular lens of claim 3, wherein said radially symmetric aberration comprises spherical aberration.

6. The intraocular lens of claim 3, wherein said radially asymmetric aberration comprises any of coma and trefoil aberrations.

7. The intraocular lens of claim 2, wherein at least one of said posterior and anterior optics (12,14) comprises another optical surface adapted to provide compensation for a third aberration type.

8. The intraocular lens of any of claims 1 to 7, wherein said selection provides a correction in a range of about - 0.5 microns to about +0.5 microns for the radially symmetric aberration.

9. The intraocular lens of any of claims 1 to 7, wherein said selection provides a correction in a range of about - 0.5 microns to about +0.5 microns for the radially asymmetric aberration.

10. The intraocular lens of claim 2, wherein said first and second optics (12,14) are axially separated by a distance in a range of about 0 to about 5 millimeters.

11. The intraocular lens of claim 2, wherein an optical axis of said posterior optic (14) is substantially aligned with an optical axis of said anterior optic (12).

12. The intraocular lens of any of claims 1 to 7, wherein said optics (12,14) are adapted to collectively provide an optical power in a range of about 6 Diopters to about 34 Diopters.

13. The intraocular lens of claim 2, wherein an index of refraction of said posterior optic (14) is different than an index of refraction of said anterior optic (12).

14. The intraocular lens of claim 2, wherein said posterior and anterior optics (12,14) have different chromatic dispersions adapted to cooperatively compensate for chromatic aberration.

15. The intraocular lens of claim 2, wherein the optic providing compensation for the radially symmetric aberration comprises a surface having a profile defined in accordance with the following relation:

$$z = \frac{cr^2}{1 + [1 - (1+k)c^2r^2]^{\frac{1}{2}}} + a_1r^2 + a_2r^4 + a_3r^6$$

wherein,

z denotes a sag of the surface at a radial distance r from an optical axis of the first optic (12),
c denotes curvature of the surface at its apex,
k denotes a conic constant,
$a_1$ denotes a second order aspheric coefficient,
$a_2$ denotes a fourth order aspheric coefficient, and
$a_3$ denotes a sixth order aspheric coefficient.

16. The intraocular lens of claim 2, wherein the optic providing compensation for the radially asymmetric aberration comprises a surface having a profile defined in accordance with the following relation:

$$z = c_{coma} * f_{coma}(r,\theta,\alpha),$$

wherein,

$$f_{coma}(r,\theta,\alpha) = 2\sqrt{3}(10r^5 - 12r^3 + 3r)\cos(\theta + \alpha)$$

wherein,

z indicates a sag of the surface along the optical axis,
$c_{coma}$ is a coefficient indicating a correction magnitude,
r is a pupil location normalized relative to the pupil radius,
$\theta$ denotes a meridian angle, and
$\alpha$ represents the coma axis to be corrected.

17. The intraocular lens of claim 16, wherein the parameter $c_{oma}$ lies in a range of about - 0.5 microns to about + 0.5 microns.

18. The intraocular lens of claim 2, wherein the optic providing compensation for the radially asymmetric aberration comprises a surface having a profile defined in accordance with the following relation:

$$z = c_{trefoil} * f_{trefoil}(r,\theta,\alpha),$$

wherein,

$$f_{trefoil}(r,\theta,\alpha) = 2\sqrt{3}(5r^5 - 4r^3)\cos(3(\theta + \alpha))$$

wherein,

$C_{trefoil}$ is a coefficient indicating a correction magnitude,
r is a pupil location normalized relative to the pupil radius,
θ is a meridian angle, and
α is the trefoil axis to be corrected.

**19.** The intraocular lens of claim 18, wherein the parameter $C_{trefoil}$ lies in a range of -0.5 microns to about +0.5 microns.

**Patentansprüche**

**1.** Mehrelement- oder Mehrflächen-Intraokularlinse (10, 22) (IOL), mit:

einer hinteren optischen Fläche (24b, 14a, 14b) und
einer vorderen optischen Fläche (24a, 12a, 12b),
wobei zumindest eine optische Fläche (24b, 14a, 14b) eingerichtet ist, um einen Aberrationstyp zu kompensieren, und zumindest eine optische Fläche (24a, 12a, 12b) eingerichtet ist, um einen anderen Aberrationstyp zu kompensieren;
**dadurch gekennzeichnet, dass** eine Kombination aus:
dem Grad an Asphärizität der optischen Flächen;
der Abbeschen Zahl des Linsenmaterials;
den Krümmungsradien der optischen Flächen;
der axialen Trennung der optischen Flächen;
dem Grad der Ausrichtung der optischen Achsen der optischen Flächen;
ausgewählt ist, um die chromatischen Längsaberrationen der Intraokularlinse (10) in Zusammenwirkung zu reduzieren oder im Wesentlichen zu beseitigen und/oder um in Zusammenwirkung eine Kompensation für die natürlichen chromatischen Aberrationen des Auges zu liefern, wodurch eine Mehrzahl von monochromatischen, polychromatischen und schiefwinkeligen Aberrationen unabhängig korrigiert werden kann und die visuelle Leistung außerhalb der optischen Achse verbessert wird, indem kundenspezifische IOLs zur Verfügung gestellt werden, die eine Kompensation für unterschiedliche Aberrationen und/oder für unterschiedliche Ausmaße der Aberrationskorrektur liefern.

**2.** Intraokularlinse nach Anspruch 1, mit:

einer hinteren Optik (14) und
einer vorderen Optik (12),
wobei die hintere Optik zumindest eine optische Fläche (14a, 14b) enthält, die eingerichtet ist, um einen Aberrationstyp zu kompensieren, und wobei die vordere Optik zumindest eine optische Fläche (12a, 12b) enthält, die eingerichtet ist, um einen anderen Aberrationstyp zu kompensieren.

**3.** Intraokularlinse nach Anspruch 1 oder 2, wobei einer der Aberrationstypen eine radial symmetrische Aberration und der andere Aberrationstyp eine radial asymmetrische Aberration umfasst.

**4.** Intraokularlinse nach Anspruch 2, wobei eine der hinteren und der vorderen Optik (12, 14) eine radial symmetrische Aberration und die andere eine radial asymmetrische Aberration kompensiert.

**5.** Intraokularlinse nach Anspruch 3, wobei die radial symmetrische Aberration sphärische Aberration umfasst.

**6.** Intraokularlinse nach Anspruch 3, wobei die radial asymmetrische Aberration eines von Koma- und Kleeblatt(Trefoil)-Aberrationen umfasst.

**7.** Intraokularlinse nach Anspruch 2, wobei zumindest eine der hinteren und der vorderen Optik (12, 14) eine weitere optische Fläche enthält, die eingerichtet ist, um einen dritten Aberrationstyp zu kompensieren.

**8.** Intraokularlinse nach einem der Ansprüche 1 bis 7, wobei die Auswahl eine Korrektur in einem Bereich von ungefähr -0,5 μm bis ungefähr +0,5 μm für die radial symmetrische Aberration liefert.

9. Intraokularlinse nach einem der Ansprüche 1 bis 7, wobei die Auswahl eine Korrektur in einem Bereich von ungefähr -0,5 μm bis ungefähr +0,5 μm für die radial asymmetrische Aberration liefert.

10. Intraokularlinse nach Anspruch 2, wobei die erste und die zweite Optik (12, 14) durch einen Abstand in einem Bereich von ungefähr 0 bis ungefähr 5 mm axial getrennt sind.

11. Intraokularlinse nach Anspruch 2, wobei eine optische Achse der hinteren Optik (14) mit einer optischen Achse der vorderen Optik (12) im Wesentlichen ausgerichtet ist.

12. Intraokularlinse nach einem der Ansprüche 1 bis 7, wobei die Optiken (12, 14) eingerichtet sind, um gemeinsam eine optische Leistung in einem Bereich von ungefähr 6 Dioptrien bis ungefähr 34 Dioptrien zu liefern.

13. Intraokularlinse nach Anspruch 2, wobei sich ein Brechungsindex der hinteren Optik (14) von einem Brechungsindex der vorderen Optik (12) unterscheidet.

14. Intraokularlinse nach Anspruch 2, wobei die hintere und die vordere Optik (12, 14) unterschiedliche chromatische Streuungen aufweisen, die eingerichtet sind, um chromatische Aberration in Zusammenwirkung zu kompensieren.

15. Intraokularlinse nach Anspruch 2, wobei die Optik, die die radial symmetrische Aberration kompensiert, eine Fläche mit einem Profil aufweist, das gemäß der folgenden Beziehung definiert ist:

$$z = \frac{cr^2}{1 + [1 - (1+k)c^2 r^2]^{\frac{1}{2}}} + a_1 r^2 + a_2 r^4 + a_3 r^6 \quad ,$$

wobei:

z eine Durchbiegung der Fläche bei einem radialen Abstand r von einer optischen Achse der ersten Optik (12) bezeichnet,
c eine Krümmung der Fläche an ihrem Scheitel bezeichnet,
k eine Kegelkonstante bezeichnet,
$a_1$ einen asphärischen Koeffizienten zweiter Ordnung bezeichnet,
$a_2$ einen asphärischen Koeffizienten vierter Ordnung bezeichnet, und
$a_3$ einen asphärischen Koeffizienten sechster Ordnung bezeichnet.

16. Intraokularlinse nach Anspruch 2, wobei die Optik, die die radial asymmetrische Aberration kompensiert, eine Fläche mit einem Profil aufweist, das gemäß der folgenden Beziehung definiert ist:

$$z = c_i * f \ (r, \theta, \alpha),$$

wobei:

$$f \ (r, \theta, \alpha) = 2\sqrt{3}(10r^5 - 12r^3 + 3r)\cos(\theta + \alpha) \quad ,$$

wobei:

z eine Durchbiegung der Fläche entlang der optischen Achse angibt,
$C_{Koma}$ ein Koeffizient ist, der eine Korrekturgröße angibt,
r eine gegenüber dem Pupillenradius normalisierte Pupillenposition ist,

θ einen Meridianwinkel bezeichnet, und
α die zu korrigierende Koma-Achse darstellt.

**17.** Intraokularlinse nach Anspruch 16, wobei der Parameter $C_{Koma}$ in einem Bereich von ungefähr -0,5 $\mu$m bis ungefähr +0,5 Mikrometer liegt.

**18.** Intraokularlinse nach Anspruch 2, wobei die Optik, die die radial asymmetrische Aberration kompensiert, eine Fläche mit einem Profil enthält, das gemäß der folgenden Beziehung definiert ist:

$$z = c \quad * f_{Trefo} \quad (r,\theta,\alpha),$$

wobei

$$f \quad (r,\theta,\alpha) = 2\sqrt{3}(5r^5 - 4r^3)\cos(3(\theta + \alpha))$$

,

wobei:

$C_{Trefoil}$ ein Koeffizient ist, der eine Korrekturgröße angibt;
r eine gegenüber dem Pupillenradius normalisierte Pupillenposition ist,
θ einen Meridianwinkel bezeichnet, und
α die zu korrigierende Trefoil-Achse darstellt.

**19.** Intraokularlinse nach Anspruch 18, wobei der Parameter $C_{Trefoil}$ in einem Bereich von -0,5 $\mu$m bis ungefähr +0,5 $\mu$m liegt.

**Revendications**

**1.** Lentille intraoculaire (IOL) à éléments multiples ou à surfaces multiples (10, 22), comprenant :

une surface optique postérieure (24b, 14a, 14b), et
une surface optique antérieure (24a, 12a, 12b),
dans laquelle au moins une surface optique (24b, 14a, 14b) est adaptée pour réaliser une compensation d'un type d'aberration et au moins une surface optique (24a, 12a, 12b) est adaptée pour réaliser une compensation d'un autre type d'aberration,
**caractérisée en ce qu'**une combinaison :
du degré d'asphéricité des surfaces optiques ;
du nombre d'Abbe du matériau de lentille ;
des rayons de courbure des surfaces optiques ;
de la séparation axiale des surfaces optiques ;
du degré d'alignement des axes optiques des surfaces optiques,
est sélectionnée pour réduire, ou éliminer sensiblement, de manière coopérative, les aberrations chromatiques longitudinales présentées par la lentille intraoculaire (10), et/ou pour réaliser de manière coopérative une compensation des aberrations chromatiques naturelles de l'oeil, moyennant quoi une pluralité d'aberrations monochromatiques, polychromatiques et obliques peuvent être corrigées de manière indépendante et les performances visuelles hors axe sont améliorées en réalisant des IOL personnalisées présentant une compensation de différentes aberrations et/ou différentes quantités de correction d'aberration.

**2.** Lentille intraoculaire selon la revendication 1, comprenant :

une optique postérieure (14), et
une optique antérieure (12),

dans laquelle ladite optique postérieure comprend au moins une surface optique (14a, 14b) adaptée pour réaliser une compensation d'un type d'aberration et ladite optique antérieure comprend au moins une surface optique (12a, 12b) adaptée pour réaliser une compensation d'un autre type d'aberration.

3. Lentille intraoculaire selon la revendication 1 ou la revendication 2, dans laquelle l'un desdits types d'aberration comprend une aberration radialement symétrique et l'autre type d'aberration comprend une aberration radialement asymétrique.

4. Lentille intraoculaire selon la revendication 2, dans laquelle l'une desdites optiques postérieure et antérieure (12, 14) réalise une compensation d'une aberration radialement symétrique, et l'autre réalise une compensation d'une aberration radialement asymétrique.

5. Lentille intraoculaire selon la revendication 3, dans laquelle ladite aberration radialement symétrique comprend une aberration sphérique.

6. Lentille intraoculaire selon la revendication 3, dans laquelle ladite aberration radialement asymétrique comprend l'une quelconque des aberrations de type coma et de type trèfle.

7. Lentille intraoculaire selon la revendication 2, dans laquelle au moins l'une desdites optiques postérieure et antérieure (12, 14) comprend une autre surface optique adaptée pour réaliser une compensation d'un troisième type d'aberration.

8. Lentille intraoculaire selon l'une quelconque des revendications 1 à 7, dans laquelle ladite sélection réalise une correction dans une plage d'environ -0,5 micron à environ +0,5 micron de l'aberration radialement symétrique.

9. Lentille intraoculaire selon l'une quelconque des revendications 1 à 7, dans laquelle ladite sélection réalise une correction dans une plage d'environ -0,5 micron à environ +0,5 micron de l'aberration radialement asymétrique.

10. Lentille intraoculaire selon la revendication 2, dans laquelle lesdites première et deuxième optiques (12, 14) sont séparées axialement d'une distance dans une plage d'environ 0 à environ 5 millimètres.

11. Lentille intraoculaire selon la revendication 2, dans laquelle un axe optique de ladite optique postérieure (14) est sensiblement aligné avec un axe optique de ladite optique antérieure (12).

12. Lentille intraoculaire selon l'une quelconque des revendications 1 à 7, dans laquelle lesdites optiques (12, 14) sont adaptées pour fournir collectivement une puissance optique dans une plage d'environ 6 dioptres à environ 34 dioptres.

13. Lentille intraoculaire selon la revendication 2, dans laquelle un indice de réfraction de ladite optique postérieure (14) est différent d'un indice de réfraction de ladite optique antérieure (12).

14. Lentille intraoculaire selon la revendication 2, dans laquelle lesdites optiques postérieure et antérieure (12, 14) ont différentes dispersions chromatiques adaptées pour compenser de manière coopérative une aberration chromatique.

15. Lentille intraoculaire selon la revendication 2, dans laquelle l'optique réalisant une compensation de l'aberration radialement symétrique comprend une surface ayant un profil défini par la relation suivants :

$$z = \frac{cr^2}{1 + [1 - (1 + k)c^2 r^2]^{\frac{1}{2}}} + a_1 r^3 + a_2 r^4 + a_3 r^6$$

z désigne un gauchissement de la surface à une distance radiale r d'un axe optique de la première optique (12),
c désigne une courbure de la surface à son sommet,
k désigne une constante de cône,

$a_1$ désigne un coefficient asphérique du deuxième ordre,
$a_2$ désigne un coefficient asphérique du quatrième ordre, et
$a_3$ désigne un coefficient asphérique du sixième ordre.

16. Lentille intraoculaire selon la revendication 2, dans laquelle l'optique réalisant une compensation de l'aberration radialement asymétrique comprend une surface ayant un profil défini par la relation suivante :

$$z = c_{coma} * f_{coma}(r, \theta, \alpha),$$

dans laquelle

$$f_{coma}(r, \theta, \alpha) = 2\sqrt{3}(10r^5 - 12r^3 + 3r)\cos(\theta + \alpha)$$

dans laquelle

z indique un gauchissement de la surface le long de l'axe optique,
$C_{coma}$ est un coefficient indiquant une amplitude de correction,
r est un emplacement de pupille normalisé par rapport au rayon de pupille,
$\theta$ désigne un angle de méridien, et
$\alpha$ représente l'axe de coma à corriger.

17. Lentille intraoculaire selon la revendication 16, dans laquelle le paramètre $C_{coma}$ est situé dans une plage d'environ -0,5 micron à environ +0,5 micron.

18. Lentille intraoculaire selon la revendication 2, dans laquelle l'optique réalisant une compensation de l'aberration radialement asymétrique comprend une surface ayant un profil défini par la relation suivante :

$$z = c_{trefoil} * f_{trefoil}(r, \theta, \alpha),$$

dans laquelle

$$f_{trefoil}(r, \theta, \alpha) = 2\sqrt{3}(5r^5 - 4r^3)\cos(3(\theta + \alpha))$$

dans laquelle

$C_{trefoil}$ est un coefficient indiquant une amplitude de correction,
r est un emplacement de pupille normalisé par rapport au rayon de pupille,
$\theta$ est un angle de méridien, et
$\alpha$ est l'axe de trèfle à corriger.

19. Lentille intraoculaire selon la revendication 18, dans laquelle le paramètre $C_{trefoil}$ est situé dans une plage de -0,5 micron à environ +0,5 micron.

13

Fig. 1

Fig. 2

Fig. 2

Fig. 3

Fig. 1

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02084381 A
- US 6416550 B
- EP 1862147 A